# FASCICULE DE BREVET EUROPEEN

(11) **EP 1 196 108 B1**
(45) Date de publication et mention de la délivrance du brevet: **21.12.2005**
(21) Numéro de dépôt: 00953237.5
(22) Date de dépôt: 13.07.2000
(51) Int. Cl.: A61B 19/00, A61B 8/00, A61B 5/00, A61B 6/00

(54) **SYSTEME TELECOMMANDABLE DE POSITIONNEMENT SUR UN PATIENT D'UN DISPOSITIF D'OBSERVATION/INTERVENTION**
AUF EINEM PATIENTEN FERNSTEUERBARES POSITIONIERUNGSSYSTEM FÜR EINE ÜBERWACHUNGS- BZW. EINGRIFFSVORRICHTUNG
REMOTELY CONTROLLABLE SYSTEM FOR POSITIONING ON A PATIENT AN OBSERVATION/INTERVENTION DEVICE

(30) Priorité: 15.07.1999 FR 9909363
(43) Date de publication de la demande: 17.04.2002
(73) Titulaire: UNIVERSITE JOSEPH FOURIER, 38041 Grenoble Cédex 9 (FR)
(72) Inventeur: CINQUIN, Philippe, F-38700 La Tronche (FR); TROCCAZ, Jocelyne, F-38320 Eybens (FR)
(74) Mandataire: de Beaumont, Michel
(86) Numéro de dépôt international: PCT/FR2000/002042
(87) Numéro de publication internationale: WO 2001/005319

(56) Documents cités:
- GB-A- 1 348 154
- US-A- 4 291 578
- US-A- 4 489 729
- US-A- 5 010 564
- US-A- 5 048 529
- US-A- 5 175 754
- US-A- 5 428 660
- US-A- 5 474 072

## Description

La présente invention concerne un système télécommandable de positionnement sur un patient d'un dispositif mobile d'observation et/ou d'intervention. Elle s'applique par exemple à des systèmes d'analyse médicale, tels que des systèmes endoscopiques ou échographiques ou à des dispositifs invasifs simples tels qu'une aiguille de ponction. Elle sera plus particulièrement décrite dans le cadre de l'utilisation d'une sonde échographique (télé-échographie).

L'échographie est une modalité d'imagerie très intéressante, car légère, inoffensive et riche en informations morphologiques et fonctionnelles. Sa réalisation demande une spécialisation particulière. Plusieurs situations cliniques requerraient la réalisation de cet examen par des moyens de télé-médecine.

La solution la plus simple, utilisée dans certaines opérations de télé-médecine, consiste pour un opérateur local à se mettre en liaison vocale et éventuellement vidéo avec un médecin expert disposé à distance. On pourrait alors imaginer que l'infirmier ou même le patient lui-même manipule une sonde échographique et que l'expert distant le guide et en tire un diagnostic. Ce recours à un expert distant est utilisé dans plusieurs situations médicales mais s'applique difficilement à l'échographie. En effet, en échographie, la réalisation et l'interprétation de l'examen sont intimement liées. Seul l'opérateur, qui a contrôlé la manière dont la sonde d'échographie a été déplacée sur le corps du patient, dispose de toutes les informations utiles pour l'interprétation. Cette caractéristique rend délicates les applications de télé-échographie. Il faut que l'opérateur local soit déjà relativement bien formé, et que l'expert distant puisse lui indiquer avec précision les déplacements de la sonde à effectuer. Or ces déplacements impliquent 6 degrés de liberté (trois translations, et trois rotations). On conçoit que l'expression par l'expert distant des ordres de déplacement sous forme vocale puisse être délicate, et plus encore leur exécution par l'opérateur local.

Pour pallier les inconvénients de la télé-échographie simple susmentionnée, il serait nécessaire de permettre à l'expert distant de prendre le contrôle du déplacement de la sonde échographique, par exemple en commandant une sonde montée sur un robot télécommandé. De tels systèmes ayant recours à la robotique sont utilisés en médecine et notamment en chirurgie. Pour cela, on utilise typiquement des architectures robotiques de type maître-esclave dans lesquelles un opérateur distant dispose d'un système à retour d'effort qui lui permet de déplacer un objet virtuel selon n degrés de liberté et dans lequel un système esclave placé à proximité du patient reproduit les mouvements du maître tandis que celui-ci peut ressentir une résistance à son déplacement.

Dans l'approche habituelle, le maître et l'esclave exécutent exactement les mêmes mouvements, l'esclave étant lié à un référentiel par rapport auquel il faut repérer la position du patient. Les contraintes mécaniques auxquelles est soumis l'esclave doivent rester dans des limites compatibles avec les possibilités de synthèse d'un retour d'effort par le maître. Par ailleurs, les architectures mécaniques utilisées font appel à des structures rigides et relativement lourdes, même lorsque la charge utile est d'un poids inférieur à 10 N. Il est donc impératif de concevoir des systèmes de sécurité performants, capables d'interdire des mouvements incontrôlés du robot, qui seraient susceptibles de blesser le patient ou l'équipe médico-chirurgicale qui l'entoure.

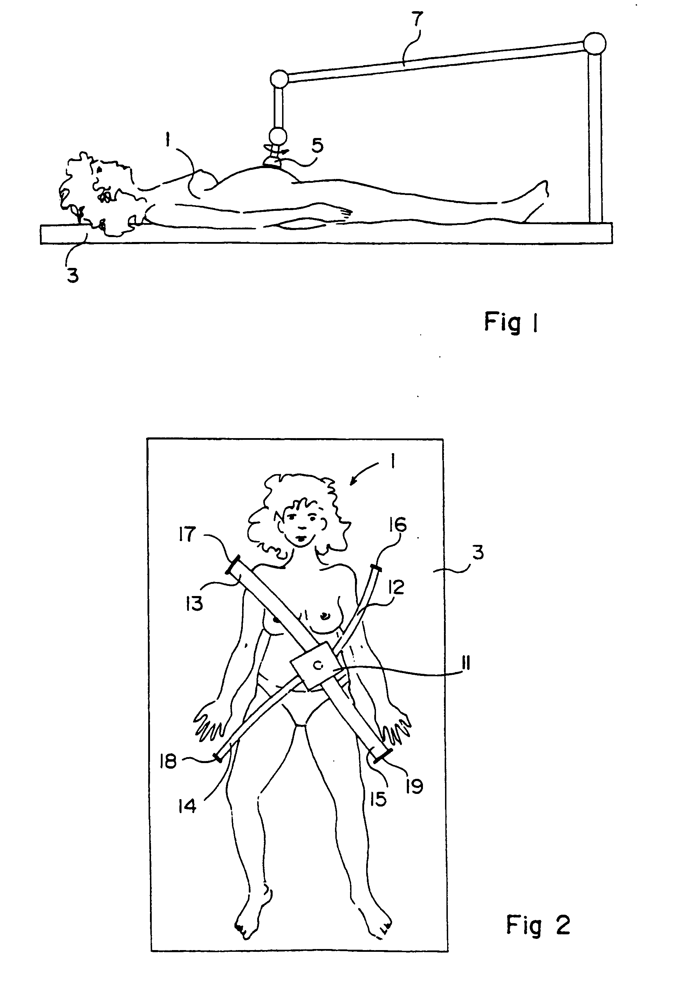

La figure 1 représente une vue de côté très schématique d'un patient 1 allongé sur une table 3 pour un examen échographique télécommandé de façon classique. Une sonde échographique 5 est disposée au contact du patient, par exemple sur son abdomen, par un système de bras de robot articulés et télécommandés 7. Un tel système implique une architecture informatique lourde pour assurer le contrôle et le retour d'effort. On notera que l'esclave (porteur de la sonde échographique) est au contact du corps humain qui exerce sur lui des pressions variables et largement imprévisibles. Ceci exige, si l'on veut que le système fonctionne en toute sécurité, la mise en oeuvre d'un système extrêmement complexe. Du fait de toutes ces contraintes, l'esclave est un système coûteux. US 4 489 726 montre un système avec des éléments à la fois souples et rigides.

On se trouve donc actuellement devant une alternative : faire appel à un opérateur local guidé par un expert distant, ce qui se révèle inadapté, ou utiliser un système robotique dont la structure mécanique et le système informatique associé sont particulièrement lourds et coûteux.

Plus généralement, le problème ci-dessus, à savoir prévoir un système de positionnement télécommandé peu coûteux et sécurisé, se pose dans de nombreux autres cas relevant ou non du secteur médical. Dans le domaine médical, un problème du même type se pose par exemple pour la télécommande en orientation et en position d'un endoscope ou d'une aiguille de ponction.

Les opérations réalisées sous endoscopie se multiplient. Elles nécessitent l'introduction de divers outils de forme globalement cylindrique à travers la peau. Le nombre de ces outils peut être tel que l'opérateur est gêné par ses assistants qui les maintiennent pour lui dans une position adéquate. Pour cette raison et d'autres, divers systèmes ont été développés pour porter et positionner des outils pénétrant le corps humain lors d'interventions sous endoscopie. Ces systèmes sont des robots "classiques", qui se fixent à la table d'opération ou au sol, et qui déplacent les outils qu'ils portent aux coordonnées qui leur sont communiquées par diverses interfaces avec l'utilisateur, voire dans certains cas par asservissement aux images observées par l'endoscope. Ces systèmes restent lourds et nécessitent une adaptation spécifique pour prendre en compte les problèmes de sécurité liés à l'utilisation de systèmes relativement rigides porteurs d'instruments chirurgicaux.

La ponction de divers organes du corps humain est un moyen très utilisé pour affiner un diagnostic (prélèvement de matériel à des fins d'analyse microscopique, mesure de diverses caractéristiques physiques, électriques en particulier), ou pour réaliser une thérapeutique (destruction physique, mécanique, chimique, électrique...). Dans de nombreux cas, cette ponction est réalisée sous le contrôle de moyens d'imagerie (radio, échographie, scanner, IRM...). Il peut être intéressant de robotiser le positionnement de l'aiguille de ponction, ce qui ouvre la voie à la réalisation automatique du geste de ponction, dans plusieurs situations cliniques, dont en particulier :
- accès au patient physiquement limité (scanner, IRM...),
- nécessité de réaliser le geste rapidement, et
- nécessité de réaliser le geste à distance.
A nouveau les systèmes de robotisation et de positionnement télécommandés existants sont trop lourds pour permettre une généralisation facile de ce type d'application.

Ainsi, un objet de la présente invention est de prévoir un système télécommandé de positionnement d'un dispositif diagnostique ou thérapeutique qui soit relativement simple.

Un autre objet de la présente invention est de prévoir un tel système qui soit peu coûteux tout en évitant tout risque pour le patient et son entourage.

Un autre objet de la présente invention est de prévoir un tel système dans lequel on puisse passer d'une commande à distance à une manipulation locale du dispositif commandé.

Un autre obj et de la présente invention est de prévoir un dispositif particulier de déplacement télécommandable d'un objet.

Pour atteindre ces objets, la présente invention prévoit de façon générale un système télécommandable de positionnement d'une charge utile, dont :
- les éléments présentent des caractéristiques de compliance, c'est-à-dire sont susceptibles de se déformer d'une manière réversible sous l'effet de contraintes mécaniques modérées, extérieures au système (ceci implique en particulier que le système ne soit pas constitué uniquement de bras rigides) ; et
- les mouvements tendent à reproduire les ordres d'un opérateur distant, mais en tenant compte de l'environnement du système, notamment de la topographie du site dans lequel est placé le dispositif dont le déplacement est télécommandé (contrôle faible), et sans qu'il soit nécessaire de connaître à tout instant les caractéristiques géométriques des divers éléments constitutifs du système.

Un tel système s'oppose donc aux robots classiques dont la plupart des éléments constitutifs sont rigides, et dans lesquels une compliance ne peut être obtenue que par des caractéristiques particulières des articulations entre les éléments rigides, et dont le contrôle implique une connaissance aussi fine que possible des caractéristiques géométriques du robot, un changeur de coordonnées permettant typiquement de passer des coordonnées "articulaires" du robot aux coordonnées cartésiennes, grâce à un modèle du robot.

Les propriétés de "compliance" et de "contrôle faible" sont particulièrement bien adaptées à des applications en médecine, lorsque l'on cherche à déplacer sur le corps humain des outils diagnostiques ou thérapeutiques. La compliance permet de mettre le système et les outils qu'il porte directement au contact du patient (dont le corps pourra même être utilisé pour contribuer à porter le système et sa charge utile). Le contrôle faible peut ne pas être un handicap ; en effet, la connaissance précise de la position du système et du dispositif commandé dans un référentiel extérieur au patient (que fournit le contrôle "classique") apporte beaucoup moins que la connaissance de la position de la charge utile par rapport aux cibles anatomiques ou thérapeutiques du corps humain. Or, cette information de position relative peut être fournie par le dispositif commandé lui-même, par divers capteurs, ou par apprentissage.

Plus particulièrement, la présente invention prévoit un système télécommandable tel que défini dans la revendication 1.

Selon un mode de réalisation de la présente invention, chacun des moyens de liaison souple est du type câble, fil ou sangle.

Selon un mode de réalisation de la présente invention, chacun des moyens de liaison souple est élastique.

Selon un mode de réalisation de la présente invention, les moyens télécommandés comprennent des moteurs enrouleurs.

Selon un mode de réalisation de la présente invention, les moyens télécommandés comprennent des muscles artificiels.

Selon un mode de réalisation de la présente invention, la liaison entre la monture et le dispositif est assurée par des moyens de liaison souple télécommandés.

Selon un mode de réalisation de la présente invention, le dispositif est une sonde échographique, et lesdits moyens de télé-observation permettent une observation de l'image échographique.

Selon un mode de réalisation de la présente invention, le dispositif est un endoscope, et les moyens de télé-observation permettent une observation de l'image endoscopique.

Selon un mode de réalisation de la présente invention, le dispositif est un porte aiguille, et les moyens de télé-observation permettent une observation d'une image de type scanner, IRM...

Selon un mode de réalisation de la présente invention, la liaison entre le patient et le centre de télécommande distant comprend une liaison audio.

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
la figure 1 représente un système de télé-échographie utilisant des techniques de robotique classiques ;
la figure 2 est une vue de dessus extrêmement schématique d'un système de télé-échographie selon la présente invention ;
la figure 3 est une vue partielle d'un exemple de monture support de sonde échographique utilisable selon la présente invention ; et
la figure 4 est une vue partielle d'un autre exemple de monture support de sonde échographique utilisable selon la présente invention.

La présente invention va d'abord être plus particulièrement décrite dans le cadre de l'utilisation d'une sonde échographique (télé-échographie).

La présente invention se base sur une analyse des besoins réels d'une opération de télé-échographie. Il faut que l'expert distant puisse :
- disposer d'une vision d'ensemble de la scène, et dialoguer avec le patient et avec le personnel local,
- contrôler les paramètres d'acquisition de l'échographe,
- visualiser les images échographiques,
- indiquer, en déplaçant une sonde virtuelle, le sens des déplacements souhaités de la sonde réelle (six degrés de liberté),
- déplacer la sonde réelle selon ces indications,
- contrôler sur les images échographiques la manière dont ses ordres de déplacement sont réellement pris en compte,
- adapter ses ordres à la manière dont ils sont réalisés et à ses besoins,
- avoir éventuellement par la sonde virtuelle un retour d'information sur la pression exercée par la sonde réelle du patient.
Il faut également que le patient ou un opérateur local puisse :
- initialiser la position du système de déplacement de la sonde,
- interrompre le déplacement de la sonde si celui-ci devient douloureux (pression trop forte, par exemple),
- redonner la main à l'expert après une telle interruption ; et
- éventuellement déplacer manuellement la sonde, selon les indications vocales de l'expert, pour faire face à des situations particulières.

La demanderesse a constaté que ces exigences pouvaient être mieux satisfaites avec un système télécommandé à structure compliante et à contrôle faible qu'avec un robot de type maître-esclave à structure rigide tel que décrit précédemment. De plus, l'utilisation d'une structure de télédéplacement souple ou compliante évite d'interdire au patient tout mouvement pendant une analyse qui, dans le cas d'une échographie, peut être relativement longue.

Selon un aspect de la présente invention, la demanderesse a noté que, en fait, quand on procède à une échographie, l'expert a une indication suffisante des mouvements que la sonde a effectué et qu'il souhaite qu'elle effectue à partir de l'image échographique qu'il reçoit. Peu importe qu'il connaisse exactement le positionnement de la sonde par rapport au patient. Il suffit que, à partir d'un positionnement donné, il puisse effectuer un déplacement situé, en gros, dans une direction (translation, rotation) souhaitée et que, après chaque déplacement incrémentiel, il puisse décider s'il souhaite continuer son déplacement dans la même direction ou se déplacer dans une autre direction pour mieux voir ce qu'il cherche à observer. Ainsi, la présente invention prévoit la suppression de toute liaison rigide entre la sonde et le support du patient. De plus, la fonction de sustentation ou de suspension de la sonde échographique est supprimée. Le système déplace la sonde sur le corps du patient tandis que celle-ci repose sur ce corps.

La figure 2 représente un mode de réalisation de la présente invention. On considère à nouveau un patient 1 allongé sur une table 3. La sonde échographique 5 est solidaire d'une monture 11 posée sur le corps du patient. Selon l'invention, on prévoit divers moyens mécaniques souples tels que des sangles, des fils, des câbles, ou analogue, pour faire glisser la monture 11 d'une sonde sur le corps du patient autour d'un emplacement choisi. Par exemple, dans le mode de réalisation représenté, la monture 11 est fixé à quatre sangles 12, 13, 14 et 15 dont les deuxièmes extrémités sont fixées à des points 16, 17, 18 et 19. Ces points 16 à 19 peuvent par exemple être des points d'attache solidaires de la table 3. Il pourra aussi s'agir de points d'attache solidaires de sangles respectivement disposées autour des bras et des cuisses du patient. On comprendra qu'en prévoyant au niveau de la liaison entre chacune des sangles et la monture un moteur enrouleur, on peut télécommander le déplacement de la monture 11 autour d'une zone initialement choisie, et sa pression sur le corps. Il sera de préférence prévu un moyen débrayable pour permettre de positionner manuellement la monture 11 dans une position initiale.

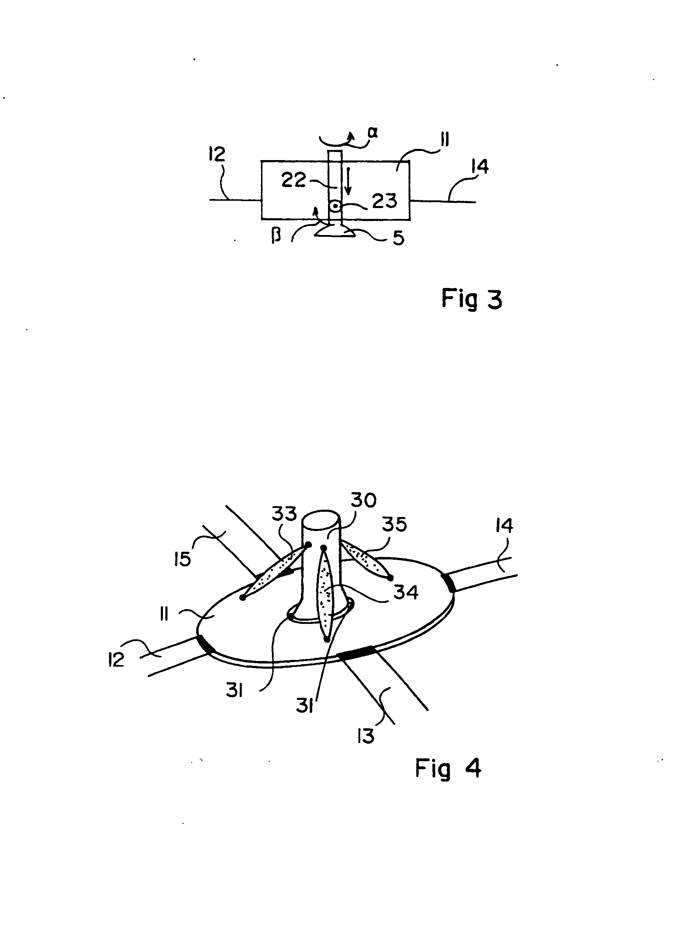

Comme le représente la figure 3, la monture 11 peut être un boîtier dans lequel la sonde échographique 21 est mobile de façon télécommandable. Par exemple, la sonde est liée à un bras 22 mobile dans le boîtier perpendiculairement à celui-ci, pour appliquer une tension plus ou moins élevée entre la sonde et le patient. Ce bras 22 sera par exemple mobile en rotation autour de son axe (direction α) et par rapport à cet axe autour d'une articulation 23 (direction β). De façon classique, on pourra prévoir plus ou moins de degrés de liberté.

Dans le mode de réalisation de la figure 4, la monture 11 est une simple plaque sur laquelle est montée, par exemple par un système à rotule 31, une sonde échographique 30. Le corps de la sonde peut être déplacé et orienté par rapport à la plaque grâce à un ensemble d'actionneurs pneumatiques 33, 34, 35. Ces actionneurs pneumatiques sont par exemple des boudins gonflables couramment appelés "muscles artificiels" dont la longueur diminue quand ils reçoivent un gaz sous pression.

Bien que cela ne soit pas décrit en détail ici, il est connu dans la technique des moyens pour asservir les divers moteurs et autres moyens télécommandables décrits précédemment aux déplacements d'un organe maître manipulé par un opérateur distant communiquant avec le système d'actionnement qui vient d'être décrit par tout moyen tel qu'une liaison radio, une liaison par câble optique, ou autre.

Divers modes d'action sur le dispositif commandé peuvent être prévus.

Le déplacement du dispositif peut être complètement automatisé et correspondre à une stratégie prédéterminée. Cette stratégie vise par exemple à satisfaire un critère de balayage complet par la sonde échographique d'un volume anatomique choisi.

Le déplacement du dispositif peut faire intervenir un expert qui s'appuie sur les images produites par le dispositif ou sur des informations physiques caractérisant le comportement du dispositif pour adapter sa stratégie médicale. Ces informations physiques peuvent notamment être :
- des informations de position, fournies par des caméras vidéo, un localisateur tridimensionnel ou des codeurs de longueur des moyens de liaison souple,
- des signaux physiologiques produits par le dispositif ou par des capteurs couplés au dispositif, ou encore
- des mesures de pression ou d'effort mécanique exercé par l'environnement sur le système.

Pour assurer la sécurité du patient, et éviter que des pressions excessives lui soient appliquées par la sonde ou sa monture, on pourra prévoir que les sangles 12-15 présentent une certaine élasticité, ou soient reliées par des systèmes à rupture. Tout autre système à sécurité passive pourra être prévu pour éviter que l'esclave n'exerce sur le patient de force ou de pression allant au-delà des limites prédéterminées. De même, les organes de déplacement entre la sonde et la monture peuvent être souples et éventuellement élastiques.

Le système de la figure 2 ne constitue qu'un exemple de réalisation de la présente invention. L'aspect de base de la présente invention est qu'elle prévoit un système permettant de télécommander le glissement et l'orientation d'un dispositif thérapeutique ou diagnostique sur le corps humain. De nombreuses variantes de réalisation peuvent être prévues pour assurer cette fonction. Par exemple, on pourra remplacer les sangles par tout autre système "à fils" éventuellement actionné par des "muscles artificiels" (boudins gonflables enserrés dans des tresses exerçant des tractions variables sous l'effet de variations de pression engendrées par des vannes susceptibles d'être commandées par ordinateur), ou par des systèmes à arceaux rigides et ressorts. Bien entendu, il faudra en outre prévoir que les divers composants du système en contact avec la peau du patient glissent sur celle-ci. On pourra par exemple, dans le cas du mode de réalisation particulier décrit, imbiber les sangles d'un gel échographique.

Il est particulièrement simple, pour un opérateur local ou pour le patient lui-même, de débrayer rapidement le système grâce à une commande facilement accessible pour interrompre le déplacement télécommandé si le système devenait douloureux, ou pour déplacer la monture de façon choisie et demandée par l'expert distant auquel le patient est lié par une liaison audio et de préférence vidéo.

Une application à un système endoscopique sera réalisée de façon analogue, le retour d'information entre l'endoscope et l'expert distant correspondant à l'image même fournie par la caméra endoscopique.

Dans le cadre d'une application de type placement d'une aiguille à ponction, le retour d'image vers l'expert distant pourra provenir d'une ou de plusieurs caméras vidéo observant l'ensemble de la scène, d'un localisateur tridimensionnel capable de suivre la position ou l'orientation de l'aiguille, ou d'un système d'imagerie particulier (rayons X, scanner, IRM...).

## Revendications

1. Système télécommandable de positionnement sur un patient d'un dispositif d'observation et/ou d'intervention comprenant :
une monture (11) à laquelle ledit dispositif est lié selon un certain nombre de degrés de liberté ;
des moyens de liaison souple (12-15) dont chacun est disposé entre la monture et un point (16-19) solidaire du support du patient ou du patient lui-même, ladite monture étant reliée auxdits points seulement par lesdits moyens de liaison souple, sans élément de guidage rigide ;
des moyens télécommandés pour modifier la longueur/tension des moyens de liaison ; et
des moyens de télé-observation du comportement du dispositif.

2. Système selon la revendication 1, **caractérisé en ce que** chacun des moyens de liaison souple est du type câble, fil ou sangle.

3. Système selon la revendication 2, **caractérisé en ce que** chacun des moyens de liaison souple est élastique.

4. Système selon la revendication 2, **caractérisé en ce que** les moyens télécommandés comprennent des moteurs enrouleurs.

5. Système selon la revendication 1, **caractérisé en ce que** les moyens télécommandés comprennent des muscles artificiels.

6. Système selon la revendication 1, **caractérisé en ce que** la liaison entre la monture et le dispositif est assurée par des moyens de liaison souple télécommandés.

7. Système selon la revendication 1, **caractérisé en ce que** ledit dispositif est une sonde échographique (5), et lesdits moyens de télé-observation permettent une observation de l'image échographique.

8. Système selon la revendication 1, **caractérisé en ce que** ledit dispositif est un endoscope, et lesdits moyens de télé-observation permettent une observation de l'image endoscopique.

9. Système selon la revendication 1, **caractérisé en ce que** ledit dispositif est un porte aiguille, et lesdits moyens de télé-observation permettent l'observation d'une image de type scanner, IRM.

10. Système selon la revendication 1, **caractérisé en ce que** la liaison entre le patient et le centre de télécommande distant comprend une liaison audio.

## Patentansprüche

1. Ein fernsteuerbares System zum Positionieren einer Überwachungs- und/oder Eingriffsvorrichtung auf einem Patienten, die Folgendes aufweist:
einen Rahmen (11) mit dem die Vorrichtung mit einer Anzahl von Freiheitsgraden verbunden ist;
flexible Verbindungsmittel (12-15), von denen jedes zwischen dem Rahmen und einem Punkt (16-19) angeordnet ist, der an der Patientenhalterung oder dem Patienten selbst befestigt ist, wobei der Rahmen mit diesen Punkten lediglich durch die flexiblen Verbindungsmittel verbunden ist, und zwar ohne irgendein starres Führungselement;
ferngesteuerte Mittel zum Modifizieren der Länge/Spannung der Bindungsmittel; und
Mittel zur Fernüberwachung des Vorrichtungsverhaltens.

2. System gemäß Anspruch 1, **dadurch gekennzeichnet, dass** jedes der flexiblen Verbindungsmittel vom Kabel-, Faden- oder Bandtyp ist.

3. System gemäß Anspruch 2, **dadurch gekennzeichnet, dass** jedes der flexiblen Verbindungsmittel elastisch ist.

4. System gemäß Anspruch 2, **dadurch gekennzeichnet, dass** die ferngesteuerten Mittel Aufwickelmotoren aufweisen.

5. System gemäß Anspruch 1, **dadurch gekennzeichnet, dass** die ferngesteuerten Mittel künstliche Muskeln aufweisen.

6. System gemäß Anspruch 1, **dadurch gekennzeichnet, dass** die Verbindung zwischen dem Rahmen und der Vorrichtung durch ferngesteuerte flexible Verbindungsmittel sichergestellt ist.

7. System gemäß Anspruch 1, **dadurch gekennzeichnet, dass** die Vorrichtung eine Echographiesonde (5) ist, und die Fernüberwachungsmittel eine Überwachung der Echographieabbildung ermöglichen.

8. System gemäß Anspruch 1, **dadurch gekennzeichnet, dass** die Vorrichtung ein Endoskop ist und die Fernüberwachungsmittel die Überwachung eines Endoskopbildes ermöglichen.

9. System gemäß Anspruch 1, **dadurch gekennzeichnet, dass** die Vorrichtung ein Nadelhalter ist und die Fernüberwachungsmittel die Überwachung einer Abbildung eines Scanner- oder Magnetresonanz-Bilddarstellungstyps (MRI) ermöglichen.

10. System gemäß Anspruch 1, **dadurch gekennzeichnet, dass** das Verbindungsglied zwischen dem Patienten und der entfernt aufgestellten Fernsteuerungszentralstation eine Sprechverbindung aufweist.

## Claims

1. A remotely controllable system for positioning on a patient an observation and/or intervention device including:
a frame (11) to which the device is bound with a number of degrees of freedom;
flexible connection means (12-15), each of which is arranged between the frame and a point (16-19) attached to the patient's support or to the patient himself, said frame being connected to said points only by said flexible connection means, without any rigid guiding element;
remotely controlled means for modifying the length/tension of the binding means; and
means for remotely observing the device behavior.

2. The system of claim 1, **characterized in that** each of the flexible binding means is of cable, thread, or strap type.

3. The system of claim 2, **characterized in that** each of the flexible binding means is resilient.

4. The system of claim 2, **characterized in that** the remotely controlled means include winder motors.

5. The system of claim 1, **characterized in that** the remotely controlled means include artificial muscles.

6. The system of claim 1, **characterized in that** the connection between the frame and the device is ensured by remotely controlled flexible binding means.

7. The system of claim 1, **characterized in that** the device is an echographic probe (5), and said remote observation means enable observation of the echographic image.

8. The system of claim 1, **characterized in that** the device is an endoscope, and the remote observation means enable observation of the endoscopic image.

9. The system of claim 1, **characterized in that** the device is a needle holder, and the remote observation means enable observation of an image of scanner or MRI type.

10. The system of claim 1, **characterized in that** the link between the patient and the distant remote-control central station includes an audio link.
